(19) Europäisches Patentamt
European Patent Office
Office européen des brevets

(11) Publication number: 0 042 705
A1

# (12) EUROPEAN PATENT APPLICATION

(21) Application number: 81302630.9

(22) Date of filing: 12.06.81

(51) Int. Cl.³: C 07 D 451/00
A 61 K 31/395

(30) Priority: 18.06.80 GB 8019936

(43) Date of publication of application:
30.12.81 Bulletin 81/52

(84) Designated Contracting States:
BE CH DE FR GB IT LI NL

(71) Applicant: BEECHAM GROUP LIMITED
Beecham House Great West Road
Brentford, Middlesex(GB)

(72) Inventor: Hadley, Michael Stewart
15 Elmwood
Sawbridgeworth Hertfordshire(GB)

(72) Inventor: Watts, Eric Alfred
217 Waterhouse Moor
Harlow Essex(GB)

(74) Representative: Dawson, Hugh Bainforde et al,
Beecham Pharmaceuticals Great Burgh Yew Tree
Bottom Road
Epsom Surrey KT18 5XQ(GB)

## (54) Azabicycloalkane derivatives, their preparation and use.

(57) Compounds of the formula (I) and pharmaceutically acceptable satls thereof:

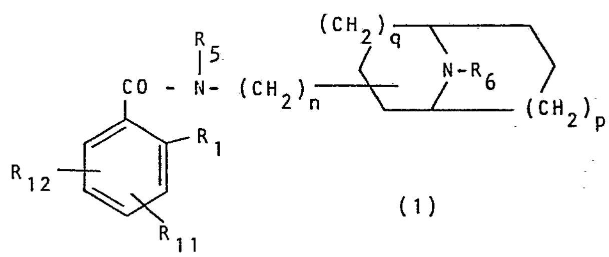

(1)

wherein:
$R_1$ is a $C_{1-6}$ alkoxy or $C_{1-6}$ alkylthio group;
$R_{11}$ is hydrogen, amino or $C_{1-7}$ acylamino;
$R_{12}$ is $C_{1-6}$ alkylsulphinyl;
$R_5$ is hydrogen or $C_{1-6}$ alkyl;
$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and n, p and q are independently 0 to 2 having useful pharmacological activity, pharmaceutical compositions containing them and process for their preparation.

EP 0 042 705 A1

TITLE MODIFIED
see front page

# ACTIVE COMPOUNDS

This invention relates to novel compounds, to pharmaceutical compositions containing them, and to a process for their preparation.

European Patent Application No 79302978.6 and allowed U.S. Patent Application No. 107,413 disclose that compounds of the formula (A), and pharmaceutically acceptable salts thereof:

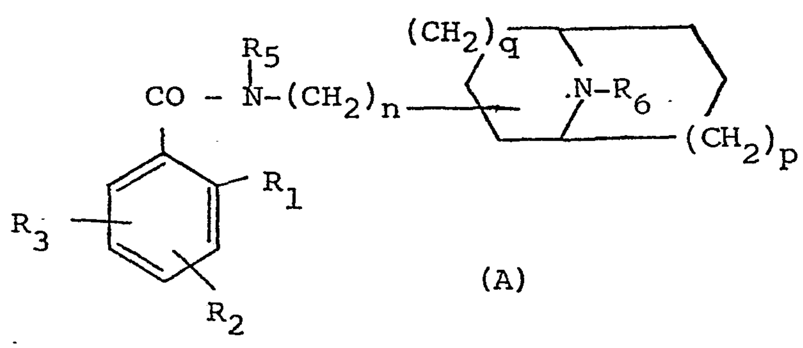

(A)

wherein:

$R_1$ is a $C_{1-6}$ alkoxy group;

$R_2$ and $R_3$ are the same or different and are hydrogen, halogen, $CF_3$, $C_{2-7}$ acyl, $C_{2-7}$ acylamino, or amino, aminocarbonyl or aminosulphone optionally substituted by one or two $C_{1-6}$ alkyl groups, $C_{1-6}$ alkylsulphone or nitro ;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen; and

n, p and q are independently 0 to 2; have useful pharmacological activity. More specifically the compounds of formula (A) are stated to be useful in the treatment of disorders related to impaired gastro-intestinal motility and/or in the treatment of disorders of the central nervous system. All the compounds are stated to have anti-emetic activity.

The said European and US Patent Applications, the subject matter of which is imported herein by reference, has extensive exemplification of typical compounds of the formula (A) and of their pharmacological activity, establishing the veracity of the claimed utilities for the class of compounds defined by formula (A).

In the said formula (A) $R_3$ may be a $C_{1-6}$ alkylsulphonyl group ($R-SO_2-$). It has now been discovered that certain compounds of a generally similar structure to that of formula (A) but wherein $R_3$ is a $C_{1-6}$ alkylsulphinyl group (R-SO-), also have useful pharmacological activity.

Accordingly the present invention provides a compound of the formula (I), and pharmaceutically acceptable salts thereof:

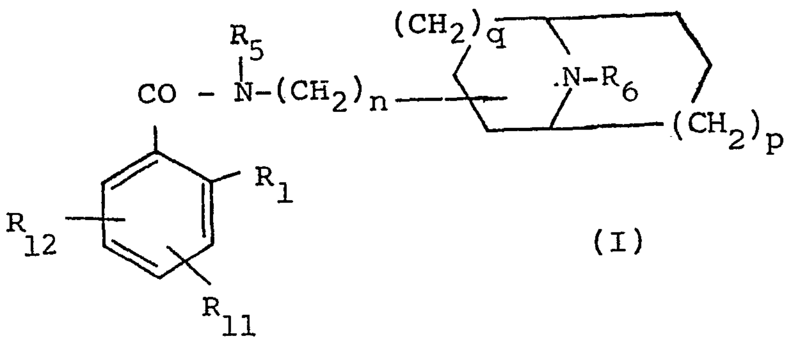

(I)

wherein:

$R_1$ is a $C_{1-6}$ alkoxy or $C_{1-6}$ alkylthio group;

$R_{11}$ is hydrogen, amino or $C_{1-7}$ acylamino;

$R_{12}$ is $C_{1-6}$ alkylsulphinyl ;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and

n, p and q are independently 0 to 2.

Examples of $R_{11}$ acylamino groups include acetylamino, propionylamino and <u>n</u> and <u>iso</u>-butyrylamino.

Examples of $R_{12}$ $C_{1-6}$ alkylsulphinyl groups include methyl, ethyl and <u>n</u> and <u>iso</u>-propyl sulphinyl.

More suitably $R_{11}$ is hydrogen, amino or acetylamino. Preferably $R_{11}$ is hydrogen or amino, most preferably amino.

Preferably $R_{12}$ is methylsulphinyl.

It is generally preferred that any $R_{11}$ group present is in the 4-position relative to the carbonyl side chain for greater activity in the resultant compound of

the formula (I). For the same reason it is generally preferred that $R_{12}$ is in the 5-position relative to the carbonyl side chain.

Thus most preferably $R_{11}$ is 4-amino and $R_{12}$ is 5-methylsulphinyl.

It will be appreciated that suitable and preferred examples of the variable groups common to formula (I) and to formula (A) will be as described in the said European and US Patent Applications. Thus:

Suitable examples of the group $R_1$ include methoxy, ethoxy and n- and iso-propoxy, methylthio, ethylthio and n- and iso-propylthio. Preferably $R_1$ is a methoxy group.

Often the amide and side chain nitrogen atoms are separated by a minimum of 2 or 3 carbon atoms, preferably 3.

When the separation is 3 atoms and n is 0, the $CONR_5$ moiety is preferably in an equatorial orientation to the bicyclic system.

Suitable examples of $R_5$ include hydrogen, methyl, ethyl, n- and iso-propyl, n-, sec- and tert-butyl, preferably hydrogen or methyl, in particular hydrogen,

Suitable examples of $R_6$ when $C_{1-7}$ alkyl include methyl, ethyl, n- and iso-propyl and n-, sec-, iso- and tert- butyl, n-pentyl, n-hexyl, n-heptyl and 3-methylbutyl.

Within $C_{1-7}$ radicals, $C_{1-4}$ alkyl are useful.

Suitable examples of $R_6$ when $C_{1-4}$ alkyl include methyl, ethyl, n- and iso-propyl and n-, sec-, iso- and tert-butyl, particularly methyl, n-propyl and sec-butyl.

Similarly, within $C_{1-7}$ radicals, $C_{5-7}$ alkyl are also of interest.

Suitable examples of $R_6$ when $C_{5-7}$ alkyl include n-pentyl, n-hexyl and n-heptyl, 3-methylbutyl, 4-methylpentyl and 5-methylhexyl.

When $R_6$ is a group $-(CH_2)_sR_7$ as defined, suitable examples of $R_7$ include $C_{5-8}$ cycloalkyl, preferably cyclohexyl. s is preferably 1.

When $R_6$ is a group $-(CH_2)_t R_8$ as defined, t is preferably 1.

In such a group $R_6$, when $R_8$ is $C_{2-5}$ alkenyl, suitable examples thereof include vinyl, prop-1-enyl, prop-2-enyl, 1-methylvinyl,but-1-enyl, but-2-enyl, but-3-enyl, 1-methylenepropyl, 1-methylprop-1-enyl and 1-methylprop-2-enyl, in their E and Z forms where stererisomerism exists.

A preferred $C_{1-5}$ alkenyl $R_8$ radical is vinyl, so that $R_6$ is preferably allyl.

When $R_8$ is optionally substituted phenyl as defined above, suitable examples of such optional phenyl substitutents include methyl, ethyl, n- and iso-propyl, n, sec- and tert-butyl; methoxy, ethoxy, n- and iso-propoxy; $CF_3$, fluoro, chloro or bromo. Preferably $R_8$ when optionally substituted phenyl is unsubstituted. When $R_8$ is thienyl it may be 2- or 3-thienyl.

Two values for $R_6$ are optionally substituted benzyl as hereinbefore defined and thienylmethyl (also called thenyl). Optionally substituted benzyl is favoured, preferably benzyl.

Compounds of the formula (I) wherein $R_6$ is $-(CH_2)_sR_7$ and $-(CH_2)_tR_8$ as defined, and wherein $R_6$ contains at least 5 carbon atoms, are of particular interest because of their beneficial pharmacological activity.

n is preferably 0. q is suitably 0 to 1, preferably 1. p is suitably 0 to 1, preferably 0.

The pharmaceutically acceptable salts of the compounds of the formula (I) include acid addition salts with conventional acids such as hydrochloric, hydrobromic, phosphoric, sulphuric, citric, tartaric, lactic and acetic acid and the like.

The pharmaceutically acceptable salts of the compounds of the formula (I) also include quaternary ammonium salts. Examples of such salts include such compounds quaternised by compounds such as $R_9$ - Y wherein $R_9$ is $C_{1-6}$ alkyl, phenyl - $C_{1-6}$ alkyl or $C_{5-7}$ cycloalkyl, and Y is an anion of an acid. Suitable examples of $R_9$ include methyl, ethyl and *n*- and *iso*-propyl; and benzyl and phenylethyl. Suitable examples of Y include the halides such as chloride, bromide and iodide.

Examples of pharmaceutically acceptable salts also include internal salts such as N-oxides.

The compounds of the formula (I) can also form hydrates, and the invention extends to such hydrates.

A group of compounds within those of the formula (I) consist of those wherein $R_{11}$ is hydrogen, amino or $C_{1-7}$ acylamino; $R_1$ is $C_{1-6}$ alkoxy;

$R_{12}$ is $C_{1-6}$ alkylsulphinyl;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen; and

n, p and q are independently 0 to 2.

From the aforesaid it will be seen that in a preferred aspect the moiety of formula (II):

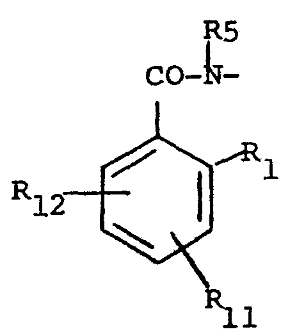

(II)

in a compound of the formula (I) will have the structure (III):

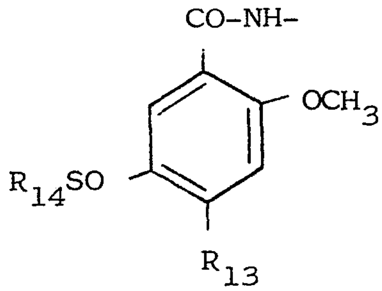

(III)

wherein $R_{14}$ is $C_{1-6}$ alkyl and $R_{13}$ is hydrogen, or amino.

Preferably $R_{13}$ is amino. Preferably $R_{14}$ is methyl or ethyl, in particular methyl.

In a preferred group of compounds within those of formula (I) and pharmaceutically acceptable salts thereof, the moiety of formula (II) will be of the formula (III), and the moiety of formula (IV):

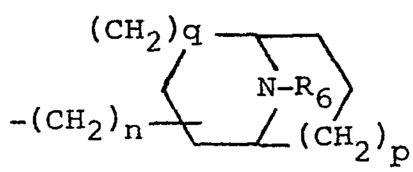

(IV)

will have the formula (V):

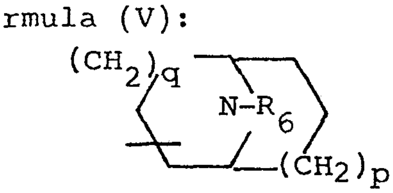

(V)

wherein the variables are as defined in formula (I), so that these preferred compounds of the formula (I) are of the formula (VI):

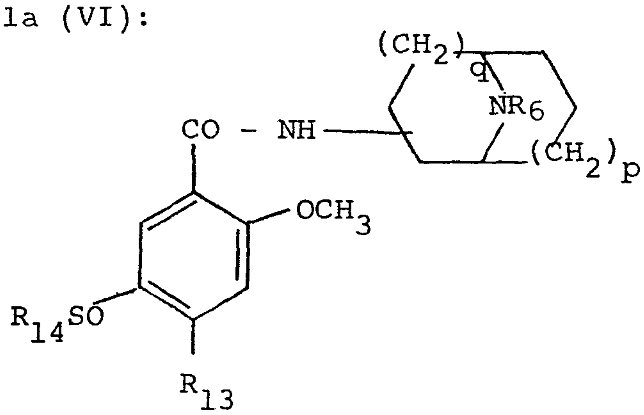

(VI)

wherein the variables are as defined above.

More suitably p is 0 or 1, it is believed preferably 0. Preferably q is 1 and the moiety of formula (III) is then attached at a position *para* to the N-atom (the 3-position; conventional numbering) and in the β orientation).

Suitable and preferred examples of $R_6$ in formula (VI) include those listed under formula (I) for $R_6$. Particularly preferred examples of $R_6$ include $C_{1-7}$ alkyl and cyclohexylmethyl. Particularly preferred examples of $R_6$ also include benzyl optionally substituted in the phenyl ring as defined under formula (I). Unsubstituted benzyl is especially preferred.

A sub-group of compounds within those of formula (VI) are those of the formula (VII):

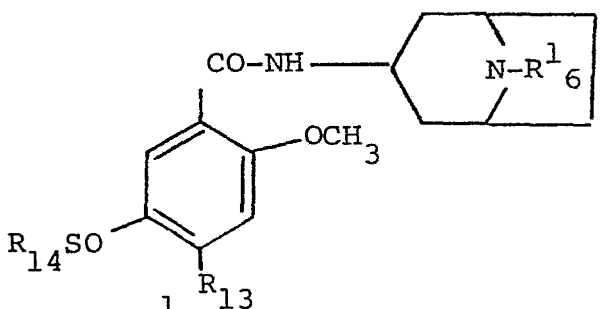

(VII)

wherein $R^1_6$ is $C_{1-4}$ alkyl.

Suitable examples of $R^1_6$ are as so described for $R_6$ $C_{1-4}$ alkyl under formula (I).

It is preferred that the moiety of the formula (III) is in the β-orientation to the nortropane ring.

A particularly preferred sub-group of compounds within those of formula (VI) are those of the formula (VIII):

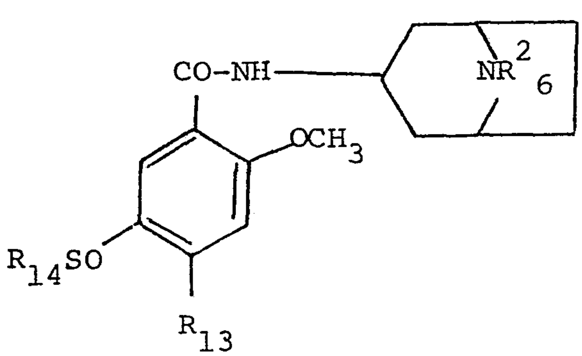

(VIII)

wherein $R^2_6$ is $C_{5-7}$ alkyl; a group $-(CH_2)_tR^1_8$ wherein t is 1 or 2 and $R^1_8$ is optionally substituted phenyl as defined in formula (I); cyclohexylmethyl, or thienylmethyl.

Suitable and preferred $R^2_6$ are as so described for the corresponding $R_6$ groups under formula (I).

$R^2_6$ benzyl is especially preferred.

It is preferred that the moiety of the formula (III) is in the β-orientation to the nortropane ring.

A sub-group of compounds within those of the formula (VI) of interest are those of the formula (IX):

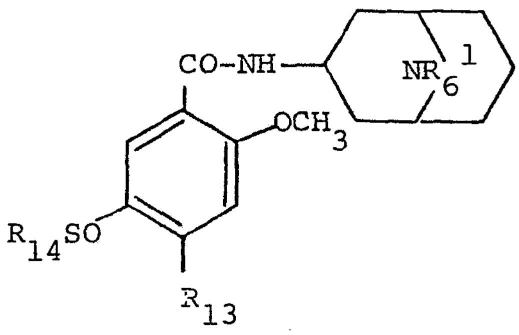

(IX)

wherein $R_6^1$ is as defined in formula (VII).

Suitable examples of $R_6^1$ are as so described under formula (VII).

Another sub-group of compounds within those of the formula (VI) of interest are those of the formula (X):

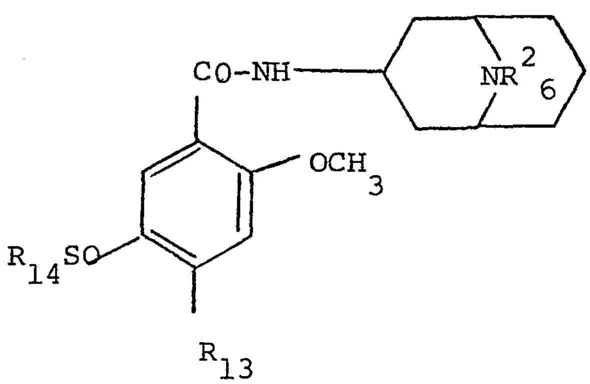

(X)

wherein $R^2_6$ is as defined in formula (VIII).

Suitable and preferred examples of $R^2_6$ are as so described under formula (VIII).

A second group of compounds within those of formula (I) is of formula (XI):

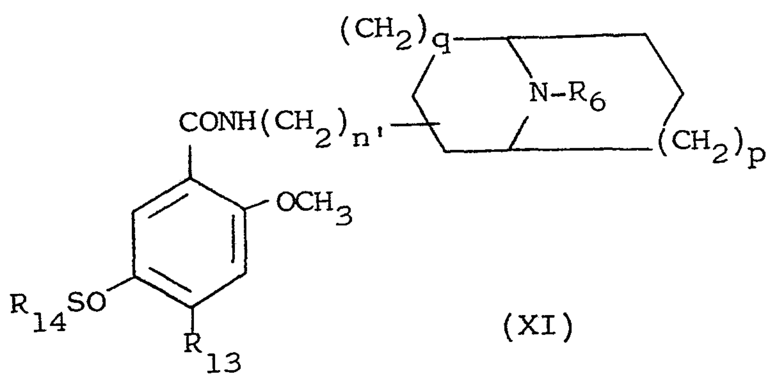

(XI)

wherein n' is 1 or 2 and the remaining variables are as defined in formula (VI).

More suitably p and q are each 0 or 1.

Preferably p is 0 and q is 1. Preferably n is 1.

Suitable and preferred $R_6$, $R_{13}$ and $R_{14}$ are as so described under formula (VI).

It will of course be realised that the compounds of the formula (I) have chiral or prochiral centres, and thus are capable of existing in a number of stereoisomeric forms. The invention extends to each of these stereoisomeri forms, and to mixtures thereof (including racemates). The different stereoisomeric forms may be separated one from the other by the usual methods, or any given isomer may be obtained by stereospecific or asymmetric synthesis.

The compounds of formula (I) may be prepared in analogous manner to the compounds of formula (A).

The invention thus provides a process for the preparation of a compound of the formula (I), which process comprises reacting an acid of the formula (XII):

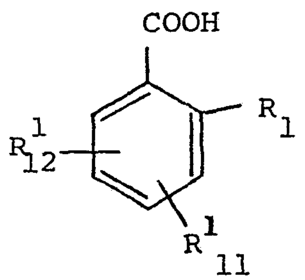

(XII)

(wherein $R^1_{11}$ is nitro, or $R_{11}$ as defined; and $R^1_{12}$ is $C_{1-6}$ alkylsulphinyl or, when $R_1$ is $C_{1-6}$ alkoxy, $C_{1-6}$ alkylthio; or a reactive derivative thereof, with a compound of formula (XIII):

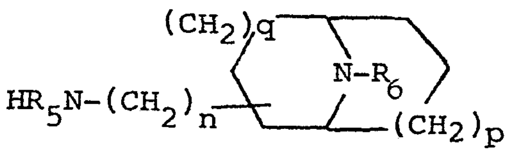

(XIII)

wherein $R_6$ is as defined in formula (I), the remaining variable groups being as defined in formula (I); and thereafter if necessary converting a group $R^1_{11}$ or $R^1_{12}$ in the thus formed compound to a group $R_{11}$ respectively; converting $R_6$ to another $R_6$; and optionally forming a pharmaceutically acceptable salt of the resultant compound of the formula (I).

'Reactive derivative' when used herein means a derivative of the compound (XII) which can be reacted with the compound (XIII) to form an amido linkage between the acid group of the compound (XII) and the amino group of

the compound of the formula (XIII).

Often this reactive derivative will be the acid halide, such as the acid chloride, of the acid (XII). In such cases, the reaction will normally be carried out in an inert solvent, preferably in the presence of an acid acceptor. The inert solvent can be any solvent inert to both reactants, such as benzene, toluene, diethyl ether or the like. The acid acceptor is suitably an organic base such as a tertiary amine e.g. triethylamine, trimethylamine, pyridine or picoline, or an inorganic acid acceptor, such as calcium carbonate, sodium carbonate, potassium carbonate or the like. It should also be noted that it is possible to use certain acid acceptors as the inert solvent, for example organic bases.

These reactions may be carried out at any non-extreme temperature such as $-10^{\circ}$-$100^{\circ}C$ and more suitably $0^{\circ}$-$80^{\circ}C$. The higher reaction temperatures are employed with less active acids of the formula (XIV) whereas the lower temperatures are employed with the more reactive acids of the formula (XIV).

Another useful reactive derivative of the acid (XII) is a highly activated ester such as the pentachlorophenyl ester, when ambient temperatures may be used. The reaction is generally effected in an inert polar solvent, such as dimethylformamide.

The reaction may also be carried out by forming an anhydride of the acid (XII) in the usual manner, and reacting that with the compound (XIII); normally a conventional mixed anhydride will be used; or by reacting the acid (XII) and the compound (XIII) in the presence of a dehydrating catalyst such as a carbodiimide, for example dicyclohexylcarbodiimide.

The intermediates of the formulae (XII) and (XIII) are either known compounds or can be prepared by analogous processes to known compounds.

It will be realised that in the compound of the formula (I) the $-CO-NR_5-(CH_2)_n-$ linkage may have an α or β orientation with respect to the ring of the bicyclic moiety to which it is attached. A mixture of α and β isomers of the compound of the formula (I) may be synthesised non-stereospecifically and the desired isomer separated conventionally therefrom, e.g. by chromatography; or alternatively the α or β isomer may if desired be synthesised from the corresponding α or β form of the compound of the formula (XIII).

Synthesis from the corresponding α or β isomer of the compound of the formula (XIII) is in general preferred.

The α or β form of the compound of the formula (XIII) may if desired be prepared by known stereospecific processes, such as those leading to the α and β isomers of the compound of the formula (XIII) depicted in the Scheme and described in Descriptions 3C, 4A and 4C of European Patent Application No. 79302978.6 and allowed U.S. Patent Application No. 107,413 for the α-isomers and Descriptions 2 and 3A and B of these Applications and present Descriptions 1 and 2A and B for the β-isomers.

The precursor of the compound of the formula (XIII) may be stereospecifically synthesised, such as the azide (D3) of Description 2 of the above European and U.S. Applications and present Description 1, and then converted to the corresponding desired isomer of the compound of the formula (XIII) under non-stereospecific conditions

with retention of configuration. Alternatively, the precursor may itself have no (pro)chiral centre at the relevant position, such as the oximes and imines of Descriptions 3 and 4 of the above European and U.S. Applications but be converted under stereospecific conditions to the desired isomer of the compound of the formula (XIII).

Alternatively, a mixture of the α and β isomers of the compound of the formula (XIII) may be synthesised non-stereospecifically and the desired isomer separated conventionally therefrom e.g. by chromatography. However, in this case it is generally more convenient to react the mixture to give a mixture of α and β isomers of the compound of the formula (I) and to separate these if desired as hereinbefore described.

The following Scheme 1 illustrates stereospecific and non-stereospecific synthetic routes to intermediates of the formula (XIII) wherein n is 0.

Scheme 1

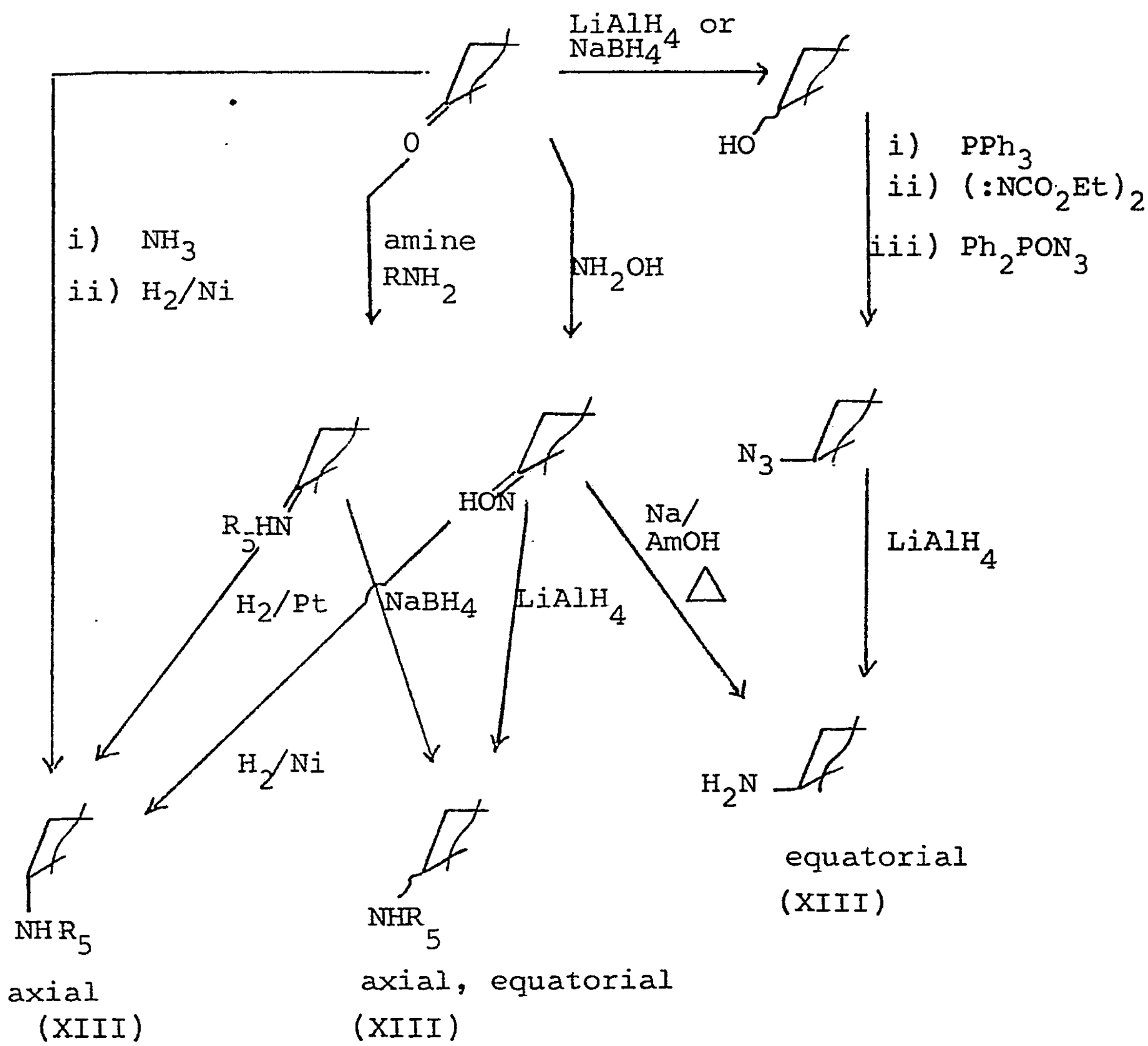


(Remainder of ring system omitted for clarity)

The following Scheme 2 illustrates preparative routes to intermediates of the formula (XIII) wherein n is 1 or 2.

Scheme 2

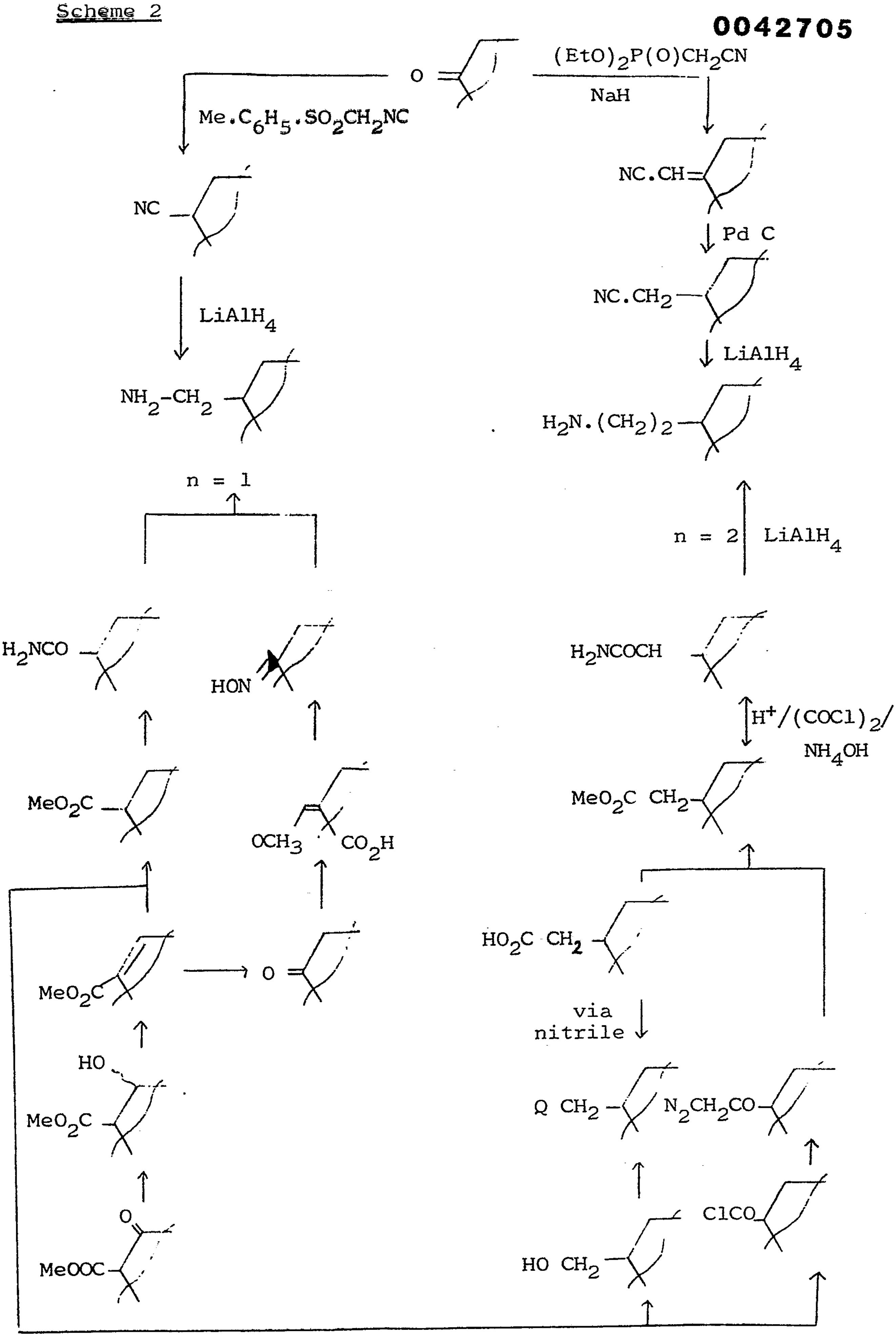
(EtO)2P(O)CH2CN
NaH
Me.C6H5.SO2CH2NC
LiAlH4
Pd C
LiAlH4
n = 1
n = 2
LiAlH4
H+/(COCl)2/
NH4OH
via
nitrile

The acid addition salts of compounds of the formula (I) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_9Y$ as defined. This reaction is suitably carried out in an appropriate solvent such as acetone, methanol, ethanol, dimethylformamide and the like, at ambient or raised temperature and pressure. The nitrogen atom of the moiety of formula (IV) may also form an N-oxide to give an internal N-oxide salt of the compound of the formula (I). The N-oxides may be prepared in conventional manner such as by reaction of the chosen compound of the formula (I) with an organic per-acid, such as m-chloroperbenzoic acid. This reaction is suitably carried out at below-ambient temperature in an organic solvent, preferably a chlorinated hydrocarbon solvent.

It will be apparent that the product of the reaction of the compounds of formulae (XII) and (XIII) will be of formula (XIV):

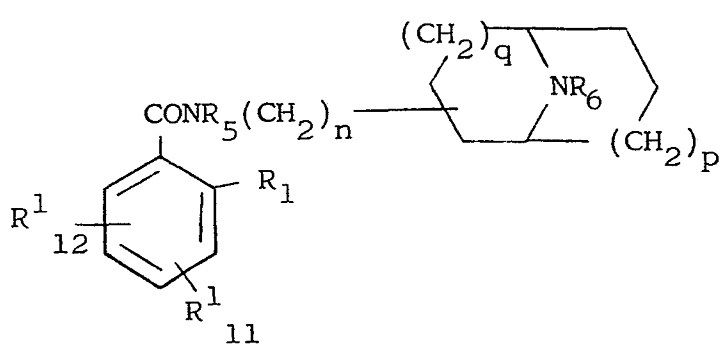

(XIV)

wherein the variables are as defined in formalae (XII) and (XIII). Compounds of the formula (XIV) containing an $R^1_{11}$ or $R^1_{12}$ group convertible to $R_{11}$ or $R_{12}$ are useful intermediates and as such form an important aspect of the invention. Some compounds of the formula (XIV) will also fall within formula (I).

Compounds of the formula (I) containing an $R_{11}$, or $R_6$ group which is convertible to another $R_{11}$, $R_6$ group are also useful intermediates and as such form an important aspect of the invention.

The skilled man will appreciate that the choice or necessity of conversion of groups $R_{11}$, $R_{11}^1$, $R_{12}^1$, or $R_6$ as above will be dictated by their nature and position.

By way of example of such conversions, the conversion of a $R^1_{11}$ nitro group to a $R_{11}$ amino group may be achieved in conventional manner such as by reduction. Thus an optional process step provided by this invention in the preparation of the compounds of the formula (I) wherein $R_{12}$ is an amino group comprises the reduction of a corresponding intermediate of formula (XIV) wherein $R_{12}$ is a nitro group.

The reduction of the intermediates wherein $R_{12}$ is a nitro group may be effected with reagents known to be suitable for reducing nitroanisole to aminoanisole. A suitable reagent for this reduction is stannous chloride in hydrochloric acid or in mixtures of hydrochloric and acetic acid. The desired amino compound may be obtained from the reaction mixture by respectively neutralisation followed by extraction into a water immiscible solvent such as ethyl acetate from which it may be recovered by evaporation of the solvent.

Another suitable method is catalytic hydrogenation at atmospheric pressure in polar solvent such as ethanol. Transition metal catalysts such as Raney nickel are often used. The desired compound may be obtained from

the reaction mixture by filtration and evaporation to dryness.

The initial crude product in both cases may be purified by chromatography or crystallisation or by forming an acid addition salt which may be recrystallised.

In general however, it is more convenient to prepare a compound of the formula (I) wherein $R_{12}$ is an amino group from the corresponding $C_{1-7}$ acylamino acid or its reactive derivative, and to deacylate the compound of the formula (I) so formed.

Those compounds of the invention wherein $R_{11}$ or $R^1{}_{11}$ is a $C_{1-7}$ acylamino group may be prepared from the corresponding intermediate wherein $R_{11}$ or $R^1{}_{11}$ is an amino group by reaction with an acylating derivative, such as previously described as a suitable acylating derivative, e.g. of the acid of the formula. The reaction may proceed as described for the reaction of the compounds of the formula. For an $R_{11}$ formamido group acylation may be effected with the free acid.

This invention thus also provides an optional process for the preparation of a compound of the formula (I) wherein $R_{11}$ is an amino group which process comprises the deacylation of a corresponding intermediate wherein $R_{11}$ or $R^1{}_{11}$ is a $C_{1-7}$ acylamino group.

Generally the hydrolysis reaction may be effected by treatment with a base such as an alkali metal hydroxide.

Although as noted $R^1{}_{11}$ may be nitro, it is generally preferred that $R^1{}_{11}$ is a $R_{11}$ group.

It will be appreciated that, when $R_6$ in the compound of the formula (XIV) is optionally substituted benzyl as hereinbefore defined, $R_6$ may be replaced by another group $R_6$.

Such $R_6$ benzyl groups may be removed by conventional transition metal catalysed hydrogenolysis to give compounds of the formula (XV):

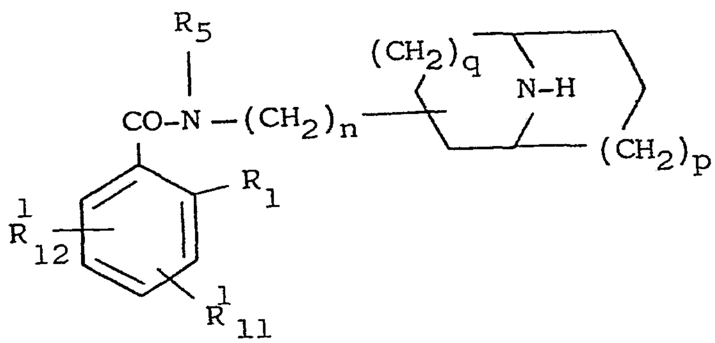

(XV)

wherein the variable groups are as defined in formulae (XII) and (XIII).

This invention also provides an optional process step in the preparation of a compound of the formula (I) which comprises the reaction of a corresponding compound of the formula (XV) as hereinbefore defined with a compound $QR_6$ wherein $R_6$ is as defined in formula (I) and Q is a group or atom readily displaced by a nucleophile, converting $R^1_{12}$ and $R^1_{11}$ in the resulting compound of formula (XIV) to $R_{12}$ and $R_{11}$ respectively and optionally forming a pharmaceutically acceptable salt of the resulting compound of the formula (I).

Suitable values for Q include Cl, Br, I, $OSO_2CH_3$ or $OSO_2C_6H_4pCH_3$.

Favoured values for Q include Cl, Br and I.

Particularly suitably the compound $QR_6$ is a benzyl halide such as benzyl bromide or benzyl chloride.

The reaction may be carried out under conventional alkylation conditions for example in an inert solvent such as dimethylformamide in the presence of an acid acceptor such as potassium carbonate. Generally the reaction is carried out at a non-extreme temperature such as at ambient or at a slightly elevated temperature.

However, it is generally more convenient to interconvert $R_6$ in the compound of the formula (XIII) before coupling with the compound of the formula (XII) or its derivative. Such interconversions are effected conveniently under the above conditions. It is desirable to protect the amine function with a group readily removable by acidolysis such as a $C_{2-7}$ alkanoyl groups before R6 interconversion.

$R^1_{12}$ $C_{1-6}$ alkylthio may be converted to $R_{12}$ as defined.

An alternative process according to the present invention thus comprises oxidising a compound of the formula (XIV) as hereinbefore defined wherein $R_1$ is $C_{1-6}$ alkoxy and $R^1_{12}$ is $C_{1-6}$ alkylthio and thereafter if necessary converting a group $R^1_{11}$ or $R_6$ in the thus formed compound to a group $R_{11}$ or $R_6$ as hereinbefore defined; and optionally forming a pharmaceutically acceptable salt of the resultant compound of the formula (I).

These oxidations may conveniently be carried out conventionally at below ambient temperatures using an organic peracid in a non- aqueous inert reaction medium preferably a chlorinated hydrocarbon solvent, for example using 3-chloroperbenzoic acid, or using a water soluble inorganic strong oxidant, such as an alkali metal permanganate, periodate or hydrogen peroxide in aqueous solution.

It will be appreciated by the skilled man that, depending on the other specific substituents in the compound of the formula (I), such an oxidation on a compound of the formula (I) may also form the N-oxide of the bicyclic moiety therein.

Given the specific substitution desired and having been decided whether the compound or its N-oxide is required, the skilled man will readily ascertain whether such interconversion is desirable.

In general however it is more convenient to prepare a compound of formula (I) from the corresponding $C_{1-6}$ alkylsulphinyl acid or its reactive derivative.

Any conversion of $R_{11}{}^{1}$, $R_{11}$, $R_{12}{}^{1}$, $R_{12}$, or $R_6$ may take place in any desired or necessary order.

The compounds of the formula (I) are dopamine antagonists.

Depending on their balance between peripheral and central action, the compounds of the formula (I) may be used in the treatment of disorders related to impaired gastro-intestinal motility, such as retarded gastric emptying, dyspepsia, flatulence, oesophagal reflux, peptic ulcer and emesis, and/or in the treatment of disorders of the central nervous system, such as psychosis.

All the compounds of the formula (I) may be used in the treatment of emesis.

The quaternary salts of the compounds of formula (I) are of interest for their beneficial effect on gastric motility.

The invention therefore also provides a pharmaceutical composition comprising a compound of the formula (I), or a hydrate or a pharmaceutically acceptable salt thereof, together with a pharmaceutically acceptable carrier. Such compositions may be adapted for oral or parenteral administration, and as such may be in the form of tablets, capsules, oral liquid preparations, powders, granules, lozenges, reconstitutable powders, injectable and infusable solutions or suspensions and the like; the compositions may also be in the form of suppositories and the like. Normally, orally administrable compositions are preferred.

Tablets and capsules for oral administration may be in unit dose presentation form, and may contain conventional excipients such as binding agents, fillers, tabletting lubricants, disintegrants, and acceptable wetting agents and the like. The tablets may be coated according to methods well known in normal pharmaceutical practice. Oral liquid preparations may be in the form of, for example, aqueous or oily suspensions, solutions, emulsions, syrups, or elixirs, or may be presented in a dry product for reconstitution with water or other suitable vehicle before use. Such liquid preparations may contain conventional additives such as suspending agents, emulsifying agents, non-aqueous vehciles (which may include edible oils), preservatives, and if desired conventional flavouring or colouring agents, and the like.

For parenteral administration, fluid unit dosage forms are prepared utilizing the compound of the formula (I) and a sterile vehicle. The compound, depending on the vehicle and concentration used, can be either suspended or dissolved in the vehicle. In preparing solutions the compound can be dissolved for injection

and filter sterilized before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to facilitate uniform distribution of the compound

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The invention further provides a method of treatment of maladies in humans comprising the administration of an effective amount of a compound of the formula (I) or a pharmaceutically acceptable salt thereof. The "effective amount" will depend in the usual way on a number of factors such as the nature and severity of the malady to be treated, the weight of the sufferer, and the actual compound used.

However by way of illustration, unit doses will suitably contain 0.01 to 20 mgs of the compound of formula (I), for example 0.02 to 10 mgs.

Again by way of illustration, such unit doses will suitably be administered more than once a day, for example 2, 3, 4, 5 or 6 times a day, in such a way that the total daily dose is suitably in the range 0.005 to 10 mg/kg per day.

Compounds of the formula (I) have the ability to potentiate the effect of conventional analgesics in migraine treatment when administered concurrently with the analgesic.

Thus the invention provides a pharmaceutical composition comprising a compound of the formula (I) and an analgesic.

The compound of the formula (I) and the analgesic, such as aspirin or paracetamol, will be present in the composition in amounts generally similar to their usual effective dose.

The composition can be a combination product, for example a tablet or capsule containing both a compound of the formula (I) and an analgesic for oral administration, or a twin pack comprising the two active ingredients made up for separate administration.

The invention accordingly provides a method of treatment of migraine comprising the administration to the sufferer of a compound of the formula (I) and an analgesic.

The following Examples illustrate the preparation of compounds of formula (I), and the following Descriptions illustrate the preparation of intermediates therefor.

Nomenclature note: Tropane is 8-methyl-8-azabicyclo [3.2.1]octane and derivatives thereof are named accordingly in the following Descriptions.

DESCRIPTION 1

3β-amino-8-benzyl-8-azabicyclo[3.2.1]octane (D1); intermediate for Compounds 1 to 3

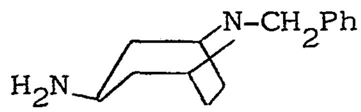

(D1)

(a) 8-benzyl-3-nortropanol (D2)

8-benzyl-3-nortropanone (3.9 g) was reduced with lithium aluminium hydride (1.0 g) in diethyl ether to 8-benzyl-3-nortropanol (D2) by the method of R.Mirza et al., Nature, 1952, 170, 630. This is claimed by Mirza to give stereospecifically the β-isomer but later workers have shown that a mixture of α and β isomers is produced.

(b) 3β-azido-8-benzylnortropane (D3)

Crude 8-benzyl-3-nortropanol (D2) (3.9 g.) was reacted with successively, triphenylphosphine (4.7 g) and diethyl azodicarboxylate (3.2 g), and diphenylphosphorylazide (5 g) in THF by the method of A.K. Bose et al., Tetrahedron Letters, 1977, 23, 1977, to yield 3β-azido-8-benzylnortropane (D3) as an oil (2 g, 25%). i.r. 2100 $cm^{-1}$ ($\nu N_3$)

(c) 3β-amino-8-benzylnortropane (D1)

To a stirred suspension of lithium aluminium hydride (0.5 g) in diethyl ether (50 ml) was added a solution of 3β-azido-8-benzylnortropane (D3) (2 g) in diethyl ether (10 ml.), and the reaction was stirred at ambient temperature for 3 hours. Hydrolysis, extraction with ethyl acetate and removal of the solvent afforded crude

3β-amino-8-benzylnortropane (1.1 g, 60%) used in the procedure of Examples 1 to 3 without further purification.

DESCRIPTION 2A

3β-amino-8-methyl-8-azabicyclo[3.2.1]octane (D4), intermediate for Compound 4

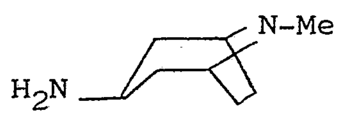

(D4)

(a) Tropinone oxime (D5)

Tropinone (3.68 g; 0.0265 mole) was dissolved in ethanol (50 ml) containing pyridine (4-5 ml) and treated with hydroxylamine hydrochloride (1.90 g). The mixture was heated under reflux for 30 minutes, cooled, treated with solid potassium carbonate (ca. 10 g) and water (ca. 5 ml). The ethanol was removed *in vacuo* and the mixture extracted with chloroform (3 x 150 ml). The combined extracts were dried ($K_2CO_3$), filtered and evaporated *in vacuo*. The resulting solid was recrystallised from ethyl acetate/petrol ether 40-60 to yield tropinone-oxime (3.1 g; 76%) as colourless crystals m.pt 114-115°C.

(b) 3β-amino-8-methyl-8-azabicyclo[3.2.1]octane (D4)

Tropinone oxime (3.08 g; 0.02 mole) was dissolved in anhydrous amyl alcohol (100 ml) and heated to almost boiling. Sodium (ca. 3.0 g) was added portionwise over 1 hour then the mixture left to cool overnight. The mixture was treated with 5N hydrochloric acid (ca. 80 ml), and extracted with ethyl acetate (3 x 150 ml). The acidic

aqueous layer was separated, basified with sodium hydroxide and re-extracted with ethyl acetate (4 x 150 ml) and the combined extracts were dried ($K_2CO_3$) filtered and evaporated in vacuo to yield (D8) (2.25 g; 80%) as a colourless oil, used without further purification.

DESCRIPTION 2B

3-β-amino-8-benzyl-8-azabicyclo[3.2.1]octane (D1); intermediate for Compounds 1 to 3

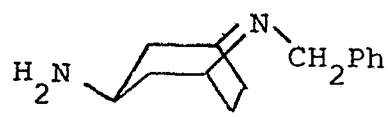

(D1)

a) N-Benzylnortropan-3-one (D.6)

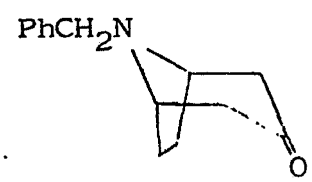

In a 500 ml r.b. flask, equipped with magnetic stirrer was placed $H_2O$ (70 ml), 2-5-dimethoxy-tetrahydrofuran (16.5 g, 0.125 mole) and 3 drops of dilute (5 N) hydroxhloric acid. When left to stir for 10 minutes the solution became clear with the formation of succindialdehyde. Benzylamine (13.65 ml, 13.4 g 0.125 mol) was mixed carefully with dilute hydrochloric acid (30 mls 5N) with cooling. When cold, this was added to the dialdehyde, followed by immediate addition of 1,3-acetonedicarboxylic acid (18.26 g, 0.125 mole) and sodium acetate (ca 10 g) in water (100 ml).

The pH of the reaction was checked to be 3-4. The reaction was sitted for 3 days at room temperature during which time carbon dioxide was evoled and the pH approached neutral.

The reaction mixture was basified to pH 8 and extracted with ethylacetate. The extracts were dried ($MgSO_4$) filtered and evaporated in vacuo. Chromatographic purification on silica (Kieselgel 60 Art 7734) using ethylacetate/light petroleum ether 40-60 gave pure N-Benzylnortropan-3-one (19.3 g, 72%) as an oil, crystallising on standing in cold.

b) N-Benzylnortropanone-Oxime (D.7)

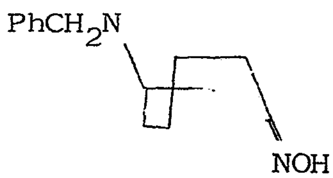

In a 500 ml r.b. flask were placed the ketone (38.40 g 0.18 mole) hydroxylamine hydrochloride (15 g, 0.22 mole) ethanol (200 ml) and pyridine (15 ml). The mixture was warmed to reflux on steam bath for 2 hours, cooled and evaporated in vacuo. The oxime was isolated by addition of water (50 ml), saturated $K_2CO_3$ and extraction with ethylacetate. The organic extracts were dried ($K_2CO_3$) filtered and evaporated in vacuo. The residue was filtered through Kieselgel 60 ethylacetate then stripped to dryness to yield the oxime (39.93 g 97%) mp. 122-124°C.

Sodium (2 g) was added portionwise over 2 hours to a stirred solution of 8-benzylnortropan-3-one

oxime (0.9 g) in amyl alcohol (20 ml) at reflux. The solution was cooled, diluted, with diethyl ether and acidified with excess dilute hydrochloric acid. The acid extract was washed with diethyl ether and then basified with excess potassium carbonate. Extraction with ethyl acetate followed by evaporation of solvent afforded crude 3β-amino-8-benzyl-nortropane (D1) (0.85 g) used in the procedure of Examples 1 to 3 without purification.

The following intermediates of the general formula

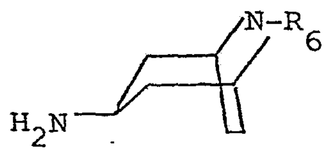

are prepared analogously:

| Intermediate No. | $R_6$ | For Compounds |
|---|---|---|
| D8 | 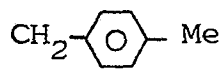 | 5,10,15 |
| D9 | $CH_2CH_2CH(CH_3)_2$ | 6,11,16 |
| D10 | $(CH_2)_3CH(CH_3)_2$ | 7,12,17 |
| D11 | $(CH_2)_4CH(CH_3)_2$ | 8,13,18 |
| D12 | 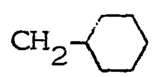 | 9,14,19 |

DESCRIPTION 3

2-Methoxy-5-methylsulphenylbenzoic acid (D.13)

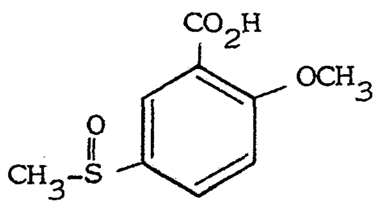

a) 5-Chlorosulphonyl-2-methoxybenzoic acid (D.14)

Chlorosulphoric acid (95.5 g, 55 ml) was added dropwise over 6 hours at a mixture of dichloroethane (72 ml), 2 methoxybenzoic acid (25 g, 0.164 mole) and dry sodium chloride (9.75 g). The mixture was warmed to 40$^{o}$ to remove most of hydrochloric acid gas, then heated at 65$^{o}$ overnight. The reaction mixture was cooled and poured into water. The resulting precipitate was filtered, washed with water and sucked as dry as possible to give 5-chlorosulphonyl-2-methoxybenzoic acid (25.65 g; 67%).

b) 2-Methoxy-5-methylthio-benzoic acid (D.15)

5-Chlorosulphonyl-2-methoxybenzoic acid (40.0 g 0.171 mole) was suspended in concentrated hydrochloric acid (100 ml) and water (50 ml). The suspension was stirred during portionwise addition of solid stannous chloride (260 g), and stirred for a further 24 hours. The resulting thick suspension was filtered and the solid was washed with dilute hydrochloric acid (100 ml) and sucked as dry as possible. This solid was dissolved in dilute sodium hydroxide solution (400 ml) and boiled. The yellow colour deepened then lightened after $^{3}/_{4}$ hour. The hot solution was filtered through Kieselguhr and treated with dimethylsulphate (18 mls). The solution was allowed to stand for 72 hours, filtered and acidified with dilute hydrochloric

acid to give 2-methoxy-5-methylthiobenzoic acid (20.20 g; 60%) as colourless microcrystals mp. 750.

c) <u>2-Methoxy-5-methylsulphinylbenzoic acid (D.13)</u>

A solution of methanol (50 ml) and 0.5 M sodium metaperiodate (52 ml) was stirred and cooled in an icebath while 2-methoxy-5-methylthiobenzoic acid (50 g, 0.0250 mole) was added over 10 minutes. After stirring at $0^{o}$ for 4 hours an additional volume of methanol (50 ml) was added and the solution was allowed to stand at room temperature for 3 days. The reaction mixture was diluted with water (100 ml) and extracted with chloroform (3 x 100 ml). The combined organic extracts were dried ($Na_2SO_4$) filtered and evaporated in vacuo to yield 2-methoxy-5-methylsulphinylbenzoic acid (2.9 g; 54%) as colourless microcrystals mp. 118-121$^{o}$.

DESCRIPTION 4

4-Acetylamino-2-methoxy-5-methylsulphinylbenzoic acid (D.16)

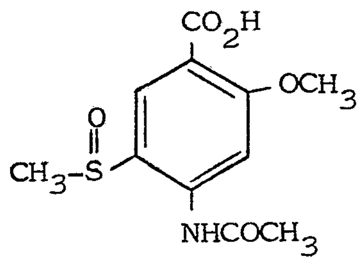

a) Methyl-4-amino-2-methoxybenzoate (D.17)

To a mixture of 4-aminosalicylic acid (60 g, 0.39 mole) and potassium hydroxide (55.2 g, 0.98 mole) in dry acetone (1200 ml) was added dimethylsulphate (87.6 ml) dropwise with vigorous stirring at room temperature. The mixture was stirred for 3 hours then the solvent was removed under reduced pressure. The solid residue was treated with water (800 ml) and filtered to give Methyl-4-amino-2-methoxy benzoate (61 g; 86%) as colourless microcrystals mp. 155-57$^{o}$ (ex ethyl acetate/petrol ether 40-60$^{o}$C).

b) Methyl-4-amino-2-methoxy-5-thiocyanobenzoate (D.18)

Methyl-4-amino-2-methoxybenzoate (18.1 g; 0.1 mole) and potassium thiocyanate (20.0 g, 0.2 mole) were dissolved in warm dry methanol (100 ml) then cooled to 0-5$^{o}$. Bromine (5.5 ml) in methanol (30 ml) was added dropwise over 1 hour. The mixture was stirred for a further hour then poured into water (2L) to give methyl-4-amino-2-methoxy-5-thiocyanobenzoate (18.8 g; 79%) as pale yellow microcrystals mp. 181-4$^{o}$C.

c) 4-Amino-2-methoxy-5-methylthiobenzoic acid (D.19)

Methyl-4-amino-2-methoxy-5-thiocyanobenzoate (35.2 g, 0.15 mole) was added to a solution of potassium hydroxide

(49.41 g) in dry methanol (1L), and the solution was heated under reflux for $^1/_2$ - $^3/_4$ hour. Methyliodide (30 ml) was added dropwise over $1^1/_2$ hour and the solution was refluxed for a further hour. The mixture was evaporated to $^1/_4$ volume and the precepitated mineral salts were filtered. The filtrate was evaporated to dryness and the residue was dissolved in water. After filtration of some methyl-4-amino-2-methoxy-5-methylthiobenzoate (0.5 g) mp. 133$^o$ the filtrate was acidified to pH5 to give 4-amino-2-methoxy-5-methylthiobenzoic acid (27 g; 85%) as colourless micro-crystals mp. 147-49$^o$.

d) <u>4-Acetylamino-2-methoxy-5-methylthiobenzoic acid (D.20)</u>

4-Amino-2-methoxy-5-methylthiobenzoic acid (4.26 g 0.02 mole) was dissolved in acetic acid (5 ml) and treated with acetic anhydride (2 ml). The mixture was warmed on water bath for 2 hours, then poured onto ice-water (ca 250 ml) to give 4-acetylamino-2-methoxy-5-methylthiobenzoic acid (3.36 g, 66%) as colourless microcrystals mp. 163-65$^o$ (ex ethylacetate/petrol 40-60$^o$).

e) <u>4-Acetylamino-2-methoxy-5-methylsulphinylbenzoic acid (D.16)</u>

4-Acetylamino-2-methoxy-5-methylthiobenzoic acid (2.0 g, 0.0078 mole) was added to a stirred solution of sodium metaperiodate (1.68 g) in methanol (50 ml) and water (50 ml). The mixture was allowed to stand at ambient temperatures overnight, poured into water (500 ml), and extracted with chloroform (3 x 100 ml). The combined organic extracts were dried ($Na_2SO_4$) filtered and evaporated in vacuo to give 4-acetylamino-2-methoxy-5-methylsulphinyl-benzoic acid (1.0 g, 47%) as colourless microcrystals mp. 193$^o$ (ex ethylacetate/ether).

DESCRIPTION 5

4-Acetylamino-5-ethylsulphinyl-2-methoxybenzoic acid

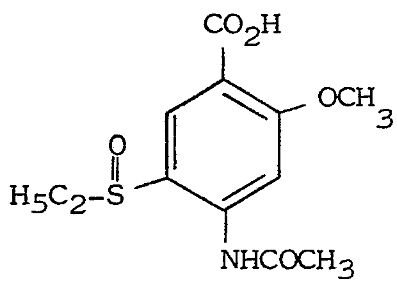

a) 4-Amino-5-ethylthio-2-methoxybenzoic acid (D.21)

Methyl-4-amino-2-methoxy-5-thiocyanobenzoate (5.40 g, 0.023 mole) was dissolved in ethanol (80 ml) containing potassium hydroxide (6.65 g) and heated to reflux for 2 hours whilst stirring. The reaction mixture was cooled to 60° and ethylbromide (bp 37-42°) (5 ml) was added dropwise. The reaction mixture was stirred at 60° for 1 hour, heated to reflux for a further hour then cooled to room temperature. The precipitated mineral salts were filtered and the filtrate was evaporated in vacuo. The residue was dissolved in water, neutralised by dropwise addition of dilute hydrochloric acid extracted into chloroform. The combined extracts were dried ($Na_2SO_4$) filtered and evaporated to dryness (5.5 g). The residue (5.5 g) was dissolved in 50% aqueous ethanol (50 ml) containing dilute sodium hydroxide (8 ml; 2.5 M) and heated to reflux for 2 hours. The mixture was evaporated in vacuo, dissolved in water (5 ml), acidified to pH 6 and extracted with chloroform (3 x 80 ml). The combined extracts were dried ($Na_2SO_4$) filtered and evaporated in vacuo to give 4-amino-5-ethylthio-2-methoxybenzoic acid (4.0 g; 77%) as a gum.

b) 4-Acetylamino-5-ethylthio-2-methoxybenzoic acid (D.23)

4-Amino-5-ethylthio-2-methoxybenzoic acid (4.0 g, 0.018 mole) was dissolved in acetic acid (15 ml), treated with acetic anhydride (2 ml) warmed on a water bath for 1 hour and left at ambiant temperatures for 18 hours. The mixture was treated with ice-water (ca 150 ml) to give 4-acetylamino-5-ethylthio-2-methoxybenzoic acid (2.8 g; 58%) mp. 134$^{O}$ (ex ethylacetate/petrol-ether 40-60$^{O}$).

c) 4-Acetylamino-5-ethylsulphinyl-2-methoxybenzoic acid (D.21)

A solution of methanol (50 ml) and water (50 ml) containing sodium metaperiodate (2.38 g) was stirred at 5-10$^{O}$ during addition of 4-acetylamino-5-ethylthio-2-methoxybenzoic acid (3.0 g, 0.0112 mole). After a further 4 hours as additional volume of methanol (50 ml) was added and the solution was allowed to stir at room temperature for 2 days. The reaction mixture was diluted with water (300 ml) and extracted with chloroform (3 x 150ml). The combined extracts were dried ($Na_2SO_4$) filtered and evaporated to give 4-acetylamino-5-ethylsulphinyl-2-methoxybenzoic acid (2.2 g; 69%) as a colourless crystalline solid mp. 171-74$^{O}$ (ex ethylacetate/ether).

Example 1

2-Methoxy-5-methylsulphinyl-N-[3β-(8-benzyl-8-azabicyclo[3,2,1]octyl)]benzamide (1)

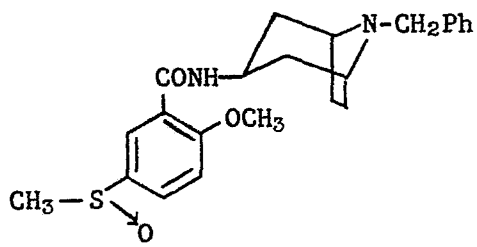

2-Methoxy-5-methylsulphinylbenzoic acid (2.8 g, 0.013 mole) was dissolved in anhydrous dimethylformamide (30 mls) and cooled to $0^O$. Triethylamine (1.32 g) was added, followed by dropwise addition of ethyl chloroformate (1.4 g) over 15 min. maintaining the temperature at $0^O$. 8-benzyl-8-azabicyclo[3,2,1]octyl-3β-amine (2.8 g) in anhydrous dimethyl formamide (20 ml) was added slowly in one portion. After 6 hours the mixture was evaporated in vacuo, the residue treated with water (15 ml) chloroform (100 ml) and basified with dilute sodium hydroxide (10 ml). The organic layer was separated, dried over sodium sulphate, filtered and evaporated in vacuo. The residue was recrystallised from ethyl acetate to give 2-methoxy-5-methylsulphinyl-N-[3β-(8-benzyl-8-azabicyclo[3,2,1]octyl)]benzamide (3.86 g) mp 165°C.

2-methoxy-5-methylsulphinyl-N-[3'β-(8'-p-methylbenzyl-8'-azabicyclo[3.2.1]octyl)]benzamide (5),
2-methoxy-5-methylsulphinyl-N-[3'β-[8'-(3"-methylbenzyl)-8'-azabicyclo[3.2.1]octyl)]benzamide (6),
2-methoxy-5-methylsulphinyl-N-[3'β-[8'-(4"-methylpentyl)-8'-azabicyclo[3.2.1]octyl)]benzamide (7),
2-methoxy-5-methylsulphinyl-N-[3'β-[8'-(5"-methylhexyl)-8'-azabicyclo[3.2.1]octyl)]benzamide (8), and

2-methoxy-5-methylsulphinyl-N-[3'β-(8'-cyclohexylmethyl-8'-azabicyclo[3.2.1]octyl]benzamide (9) are prepared analogously.

Example 2

4-Amino-2-methoxy-5-methylsulphinyl-N-[3'β-(8'-benzyl-8'-azabicyclo[3,2,1]octyl)]benzamide (2)

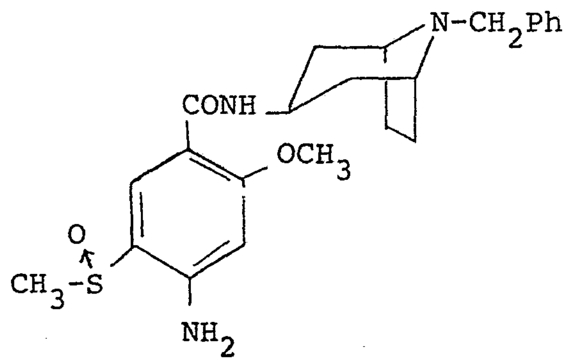

4-Acetylamino-2-methoxy-5-methylsulphinyl benzoic acid (0.94 g, 0.0035 mole) (as prepared in GB 2,013,662A) was dissolved in dry dimethylformamide (20 ml) containing triethylamine (0.35 g) and cooled to $0^{o}C$. Ethyl chloroformate (0.38 g) was added dropwise so that the temperature remained between $0^{o}$ and $3^{o}$. 8-Benzyl-8-azabicyclo[3,2,1]octyl-3β-amine (0.75 g, 0.0035 mole) in dry dimethylformamide (5 ml) was added after 10 minutes. The reaction mixture was left for 24 hours, evaporated to dryness, treated with dilute sodium hydroxide (10 ml; 10%) and extracted with ethyl acetate (3 x 100 ml). The combined organic extracts were dried ($K_2CO_3$) filtered and evaporated to dryness to give 4-acetylamino-2-methoxy-5-methylsulphinyl-N-[3'β-(8'-benzyl-8'-azabicyclo{3,2,1}octyl)]benzamide as a glass (1.0 g). This glass was treated with ethanol (10 ml) and water (1 ml) containing 85% potassium hydroxide (0.45 g) and warmed to $80^{o}$ for 2 hours. The mixture was cooled, evaporated to dryness, treated with water (10 ml) and extracted with ethyl acetate

(3 x 100 ml). The combined organic extracts were dried ($K_2CO_3$), filtered and evaporated in vacuo. Treatment of the residue with dry ether gave 4-amino-2-methoxy-5-methylsulphinyl-N-[3β-(8'-benzyl-8'-azabicyclo{3,2,1}octyl)] benzamide (0.55 g, 40%), mp 189-190° containing 20% 4-amino-2-methoxy-5-methylsulphonyl-N-[3β-(8'-benzyl-8'-azabicyclo{3,2,1}octyl)] benzamide.

Recrystallisation from ethyl acetate/light petroleum ether 40-60 gave the sulphine (0.26 g, 18%), mp 194-195° containing about 10% of the sulphone.

Nmr

($\delta$, $CDCl_3$) : (2) 8.02 (s, 1H, aryl 6H);
7.6-7.1 (m, 6H, Ph-H and CONH);
6.2 (s, 1H, aryl, 3-H);
5.71 (m, 2H, $-NH_2$);
4.6-4.1 (m, 1H, CONH-CH<);
3.89 (s, 3H, $OCH_3$);
3.53 (s, 2H, $PhCH_2$-N<);
3.24-3.22 (m, 2H, $(PhCH_2)N(CH<)_2$);
2.87 (s, 3H, $CH_3SO$);
2.3-1.4 (m, 8H, $>CH_2$).

corresponding sulphone
8.60 (s, 1H, aryl, 6H);
6.17 (s, 1H, aryl, 3H);
3.84 (s, 3H, $OCH_3$);
2.98 (s, 3H, $CH_3SO_2$).

4-amino-2-methoxy-5-methylsulphinyl-N-[3'β-(8'-p-methylbenzyl-8'-azabicyclo[3.2.1]octyl)]benzamide (11),
4-amino-2-methoxy-5-methylsulphinyl-N-[3'β-[8'-(3"-methylbutyl-8'-azabicyclo[3.2.1]octyl)]benzamide (12),
4-amino-2-methoxy-5-methylsulphinyl-N-[3'β-[8'-(4"-methylpentyl)-8'-azabicyclo[3.2.1]octyl)]benzamide (13),
4-amino-2-methoxy-5-methylsulphinyl-N-[3'β-[8'-(5"-methylhexyl)-8'-azabicyclo[3.2.1]octyl)]benzamide (14) and,

4-amino-2-methoxy-5-methylsulphinyl-N-[3'β-[8'-cyclohexylmethyl-8'-azabicyclo[3.2.1]octyl)]benzamide (15) are prepared analogously.

Example 3

4-Amino-5-ethylsulphinyl-2-methoxy-N-[3'β-(8'-benzyl-8'-azabicyclo{3,2,1}octyl)]benzamide (3)

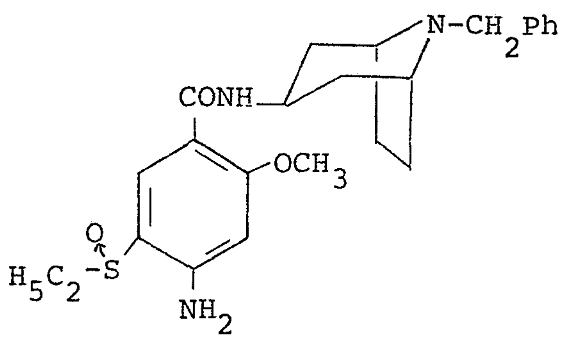

4-Acetylamino-5-ethylsulphinyl-2-methoxy-benzoic acid (2.2 g, 0.0077 mole) (prepared as described in GB 2,013,662A) was dissolved in dry dimethylformamide (30 ml) containing triethylamine (0.78 g, 1.08 ml) and cooled to $0^{o}$. Ethyl chloroformate (0.84 g, 0.75 ml) was added dropwise at $0^{o}$. After a further 10 minutes at $0-3^{o}$, 8-benzyl-8-azabicyclo[3,2,1]octyl-3-amine (1.67 g) was added in dry dimethylformamide (8 ml) and the mixture was left to reach ambient temperatures over 24 hours. The mixture was evaporated to dryness, treated with water (10 ml), dilute sodium hydroxide (10 ml, 10%) and extracted with chloroform (3 x 100 ml). The combined organic extracts were dried ($Na_2SO_4$) filtered and evaporated to dryness. The residue was chromatographed on Kieselgel 60 with ethyl acetate as eluant to give 4-acetylamino-5-ethylsulphinyl-2-methoxy-N-[3'β-(8'-benzyl-8'-azabicyclo{3,2,1}octyl)] benzamide (1.86; 50%) as a foam.

Hydrolysis as described in Example 2 gave 4-amino-5-ethylsulphinyl-2-methoxy-N-[3'β-(8'-benzyl-8'-azabicyclo{3,2,1}octyl)] benzamide (1.0 g, 29%) as colourless microcrystals, mp 169-170° ex ether/ethyl acetate. $C_{24}H_{31}N_3O_2S$; Observed $M^+$ = 441.2081 Calculated $M^+$ = 441.2084. Required %C = 65.31, H = 7.03; N = 9.52. Found %C = 65.06; H = 7.32; N = 9.58.

4-amino-2-methoxy-5-ethylsulphinyl-N-[3'β-(8'-p-methylbenzyl-8'-azabicyclo[3.2.1]octyl)]benzamide (16), 4-amino-2-methoxy-5-ethylsulphinyl-N-(3'β-[8'-(3"-methylbutyl)-8'-azabicyclo[3.2.1]octyl)]benzamide (17), 4-amino-2-methoxy-5-ethylsulphinyl-N-[3'β-[8'-p-(4"-methylpentyl)-8'-azabicyclo[3.2.1]octyl)]benzamide (18), 4-amino-2-methoxy-5-ethylsulphinyl-N-{3'β-[8'-(5"-methylhexyl)-8'-azabicyclo[3.2.1]octyl]}benzamide (19), and 4-amino-2-methoxy-5-ethylsulphinyl-N-[3'β-(8'-cyclohexylmethyl-8'-azabicyclo[3.2.1]octyl) are prepared analogously.

Example 4

4-Acetylamino-2-methoxy-5-methylsulphinyl-N-[3'β-(8'-methyl-8'-azabicyclo{3,2,1}octyl)]benazmide (4)

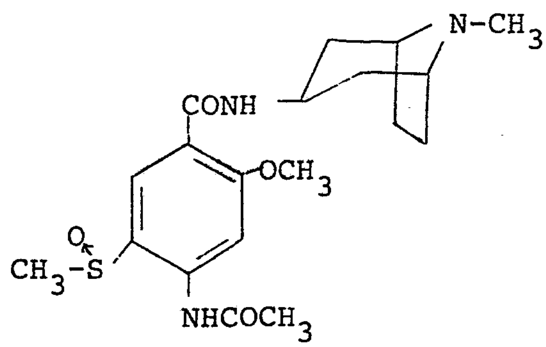

4-Acetylamino-2-methoxy-5-methylsulphinyl benzoic acid (0.63 g, 0.0023 mole) was dissolved in dry dimethylformamide (15 ml) containing triethylamine (0.23 g, 0.32 ml), cooled to 0°, and treated dropwise with ethyl chloroformate (0.25 g, 0.22 ml). After 10 minutes, 8-methyl-8-azabicyclo[3,2,1]octyl-3β-amine (0.33 g) in dry dimethylformamide (5 ml) was added and the mixture left 24 hours. The mixture was evaporated to dryness, treated with $H_2O$ (10 ml), dilute sodium hydroxide (10 ml, 10%) and extracted with chloroform (3 x 100 ml). The combined organic extracts were dried ($K_2CO_3$), filtered and evaporated to dryness. The residue was triturated with dry ether and the resulting colourless microcrystals (0.70 g, 77%), mp 199-200°C recrystallised from ethylacetate to give 4-acetylamino-2-methoxy-5-methylsulphinyl-N-[3'β-(8'-methyl-8'azabicyclo{3,2,1}octyl)] benzamide (0.60 g, 66%) mp 199-200°C.

$C_{19}H_{27}N_3O_4S$ . Observed $M^+$ = 393.1729; Calculated $M^+$ 393.1722

| | | |
|---|---|---|
| 1H Nmr ($CDCl_3$) | : | 11.20 (m, 1H, -N$\underline{H}$COC$H_3$); |
| δ, ppm | | 8.40 (s, 1H, aryl 6-H); |
| | | 8.06 (s, 1H, aryl 3-H); |
| | | 3.97 (s, 3H, $OCH_3$); |
| | | 2.89 (s, 3H, C$\underline{H}_3$-S→O); |
| | | 2.29 (s, 3H {NHCOC$\underline{H}_3$} and |
| | | 2.21 (s, 3H {N-$CH_3$} |

## Pharmacological Activity

### Anti-emetic Activity in the Dog

The compounds were administered subcutaneously 30 minutes prior to administration of a standard dose of apomorphine HCl (0.1 mg/kg subcutaneously) and the vomiting response compared to that obtained when the same animals were dosed with apomorphine HCl and vehicle only. The dose that totally inhibited the vomiting response in 50% or more the dogs was determined.

| Compound | Anti-emetic Activity Active dose mg/kg subcutaneously |
|---|---|
| 1 | 0.1 |
| 2 | 0.002* |
| 3 | 0.01 |

* Activity in excess to that which can be accounted for by the 10% contamination of the corresponding sulphone.

### Toxicity

No toxic effects were observed in the above tests.

Claims:

1. A compound of the formula (I) or a pharmaceutically acceptable salt thereof:

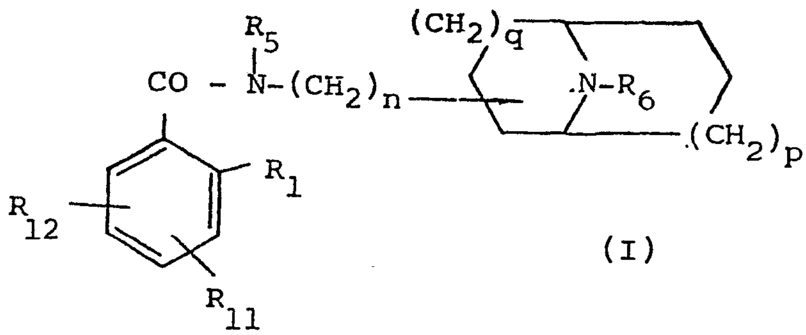

(I)

wherein:

$R_1$ is a $C_{1-6}$ alkoxy or $C_{1-6}$ alkylthio group;

$R_{11}$ is hydrogen, amino or $C_{1-7}$ acylamino;

$R_{12}$ is $C_{1-6}$ alkylsulphinyl;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ aklenyl or a phenyl group optionally substituted by one or two substituents selected from $C_{1-6}$ alkyl, $C_{1-4}$ alkoxy, trifluoromethyl and halogen, or a thienyl group; and

n, p and q are independently 0 to 2.

2. A compound according to claim 1 or a pharmaceutically acceptable salt thereof, characterised in that: $R_1$ is $C_{1-6}$ alkoxy;

$R_{11}$ is hydrogen, amino or $C_{1-7}$ acylamino;

$R_{12}$ is $C_{1-6}$ alkylsulphinyl;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen; and

n, p and q are independently 0 to 2.

3. A compound according to claim 1 or 2 of formula (VI) or a pharmaceutically acceptable salt thereof:

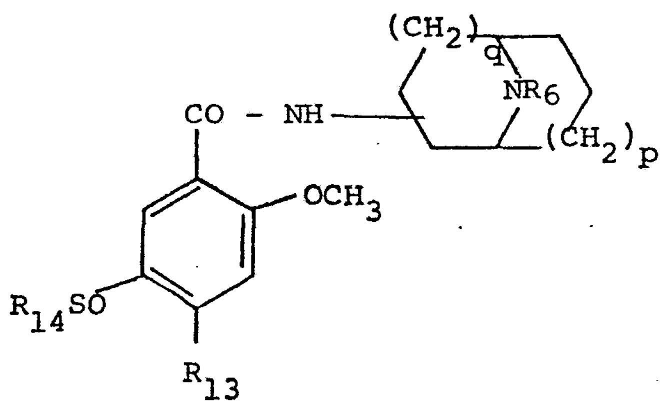

(VI)

characterised in that $R_{13}$ is hydrogen or amino and $R_{14}$ is $C_{1-6}$ alkyl.

4. A compound according to claim 3 of formula (VIII) or a pharmaceutically acceptable salt thereof:

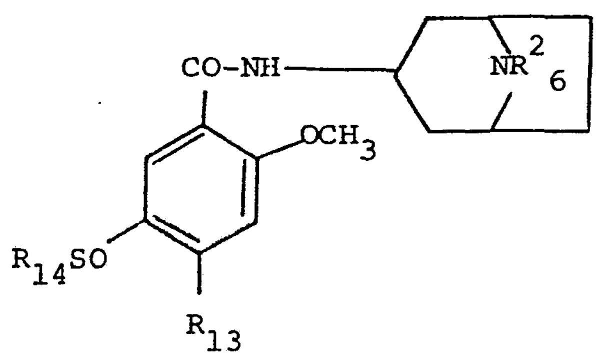

(VIII)

characterised in that:

$R^2_6$ is $C_{5-7}$ alkyl;

a group $-(CH_2)_tR^1_8$ characterised in that t is 1 or 2 and $R^1$ is optionally substituted phenyl as defined in claim 1; cyclohexylmethyl, or 2-thienylmethyl and

$R_{13}$ and $R_{14}$ are as defined in claim 3.

5. 2-Methoxy-5-methylsulphinyl-N-[3β-(8-benzyl-8-azabicyclo[3,2,1]octyl)]benzamide,
2-methoxy-5-methylsulphinyl-N-[3'β-(8'-p-methylbenzyl-8'-azabicyclo[3.2.1]octyl)]benzamide,
2-methoxy-5-methylsulphinyl-N-[3'β-[8'-(3"-methylbenzyl)-8'-azabicyclo[3.2.1]octyl)]benzamide,
2-methoxy-5-methylsulphinyl-N-[3'β-[8'-(4"-methylpentyl)-8'-azabicyclo[3.2.1]octyl)]benzamide,
2-methoxy-5-methylsulphinyl-N-[3'β-[8'-(5"-methylhexyl)-8'-azabicyclo[3.2.1]octyl)]benzamide,
2-methoxy-5-methylsulphinyl-N-[3'β-(8'-cyclohexylmethyl-8'-azabicyclo[3.2.1]octyl)]benzamide,

4-Amino-2-methoxy-5-methylsulphinyl-N-[3'β-(8'-benzyl-8'-azabicyclo[3.2.1]octyl)]benzamide,
4-amino-2-methoxy-5-methylsulphinyl-N-[3'β-(8'-p-methylbenzyl-8'-azabicyclo[3.2.1]octyl)]benzamide,
4-amino-2-methoxy-5-methylsulphinyl-N-[3'β-[8'-(3"-methylbutyl-8'-azabicyclo[3.2.1]octyl)]benzamide,
4-amino-2-methoxy-5-methylsulphinyl-N-[3'β-[8'-(4"-methylpentyl)-8'-azabicyclo[3.2.1]octyl)]benzamide,
4-amino-2-methoxy-5-methylsulphinyl-N-[3'β-[8'-(5"-methylhexyl)-8'-azabicyclo[3.2.1]octyl)]benzamide,
4-amino-2-methoxy-5-methylsulphinyl-N-[3'β-[8'-cyclohexylmethyl-8'-azabicyclo[3.2.1]octyl)]benzamide,

4-Amino-5-ethylsulphinyl-2-methoxy-N-[3'β-(8'-benzyl-8'-azabicyclo[3.2.1]octyl)]benzamide,
4-amino-2-methoxy-5-ethylsulphinyl-N-[3'β-(8'-p-methylbenzyl-8'-azabicyclo[3.2.1]octyl)]benzamide,
4-amino-2-methoxy-5-ethylsulphinyl-N-(3'β-[8'-(3"-methylbutyl)-8'-azabicyclo[3.2.1]octyl)benzamide,

4-amino-2-methoxy-5-ethylsulphinyl-N-[3'β-[8'-p-(4"-methylpentyl)-8'-azabicyclo[3.2.1]octyl)]benzamide,
4-amino-2-methoxy-5-ethylsulphinyl-N- 3'β-[8'-(5"-methylhexyl)-8'-azabicyclo[3.2.1]octyl[ benzamide,
4-amino-2-methoxy-5-ethylsulphinyl-N-[3'β-(8'-cyclohexylmethyl-8'-azabicyclo[3.2.1]octyl.
or a pharmaceutically acceptable salt thereof.

6. A compound according to claim 3 or a pharmaceutically acceptable salt thereof of the formula (X):

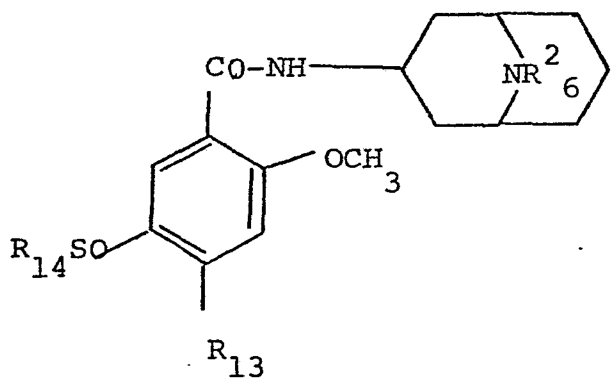

(X)

wherein:

$R^2_6$ is as defined in claim 4.

7. A process for the preparation of a compound of the formula (I) according to claim 1, characterised by

a) reacting an acid of the formula (XII):

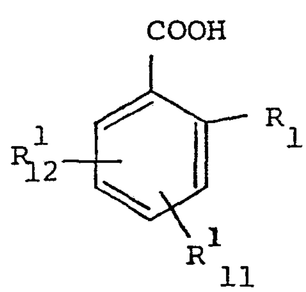

(XII)

(wherein $R^1_{11}$ is nitro, or $R_{11}$ as defined; and

$R^1_{12}$ is $C_{1-6}$ alkylsulphinyl or, when $R_1$ is $C_{1-6}$ alkoxy, $C_{1-6}$ alkylthio; or a reactive derivative thereof, with a compound of formula (XIII):

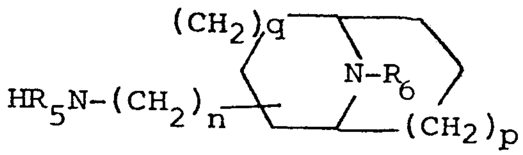

(XIII)

wherein $R_6$ is as defined in formula (I), the remaining variable groups being as defined in formula (I); and thereafter if necessary converting a group $R^1_{11}$ or $R^1_{12}$ in the thus formed compound to a group $R_{11}$ respectively; optionally converting $R_6$ to another $R_6$; and optionally forming a pharmaceutically acceptable salt of the resultant compound of the formula (I) or

b) reacting a compound of the formula (XV) or a salt thereof:

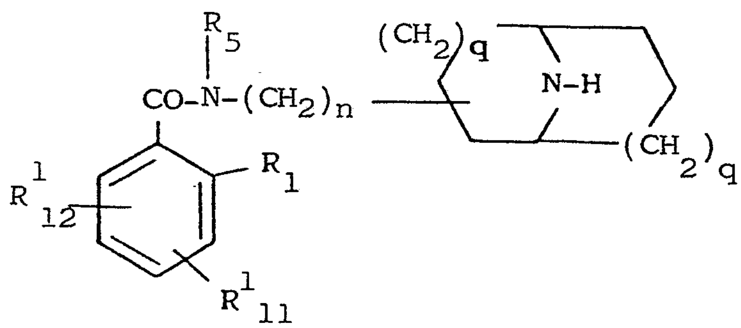

(XV)

wherein:

the variable groups are as defined in formulae (XII) and (XIII) with a compound $QR_6$ wherein $R_6$ is as defined in formula (I) and Q is a group or atom readily displaced by a nucleophile, converting $R^1_{12}$ and $R^1_{11}$ in the resulting compound to $R_{12}$ and $R_{11}$ as defined in formula (I) respectively, and optionally forming a pharmaceutically acceptable salt of the resultant compound of the formula (I).

8. A pharmaceutical composition comprising a compound according to claim 1 or a hydrate or a pharmaceutically acceptable salt thereof, together with a pharmaceutically acceptable carrier.

9. A method of treatment of maladies in humans comprising the administration of a therapeutically effective amount of a compound according to claim 1 or a pharmaceutically acceptable salt thereof.

0042705

Application number

EP 81302630.9

European Patent Office

# PARTIAL EUROPEAN SEARCH REPORT

which under Rule 45 of the European Patent Convention shall be considered, for the purposes of subsequent proceedings, as the European search report

## DOCUMENTS CONSIDERED TO BE RELEVANT

| Category | Citation of document with indication, where appropriate, of relevant passages | Relevant to claim |
|---|---|---|
| | US - A - 2 800 483 (JUCKER)<br>+ Columns 1,2 +<br>-- | 1-4, 6-8 |
| | DE - A1 - 2 645 781 (BASF AG)<br>+ Pages 3-7,9 +<br>-- | 1-4,6 |
| P | & GB-A-1 586 793 (25-03-1981)<br>---- | |

CLASSIFICATION OF THE APPLICATION (Int. Cl.[3])

C 07 D 451/00
A 61 K 31/395

TECHNICAL FIELDS SEARCHED (Int. Cl.[3])

C 07 D 451/00
C 07 D 471/00
C 07 D 487/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8
Claims searched incompletely: -
Claims not searched: 9
Reason for the limitation of the search:

Method for treatment of the human or animal body by therapy, article 52(4) EPC

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| VIENNA | 25-08-1981 | PETROUSEK |